# EUROPEAN PATENT SPECIFICATION

(11) **EP 3 952 828 B1**
(45) Date of publication and mention of the grant of the patent: **07.05.2025**
(21) Application number: 20787828.1
(22) Date of filing: 09.04.2020
(51) Int. Cl.: A61K 8/24, A61K 8/27, A61K 8/34, A61K 8/20, A61K 8/73, A61Q 11/00

(54) **ORAL RINSE COMPOSITIONS FOR REDUCING PATHOGENS UNDERLYING TOOTH DECAY, PERIODONTITIS AND ORAL MALODOR**
MUNDSPÜLUNGSZUSAMMENSETZUNGEN ZUR REDUZIERUNG VON PATHOGENEN, DIE KARIES, PARODONTITIS UND MUNDGERUCH ZUGRUNDE LIEGEN
COMPOSITIONS DE RINÇAGE BUCCAL POUR RÉDUIRE LES PATHOGÈNES RESPONSABLES DE LA CARIE DENTAIRE, DE LA PARODONTITE ET DE LA MAUVAISE HALEINE

(30) Priority: 10.04.2019 US 201962832107 P
(43) Date of publication of application: 16.02.2022
(73) Proprietor: Black Tulip Management, Inc., New York, NY 10016 (US)
(72) Inventor: SCALA, Anthony, Jamesville, NY 13078 (US); MALPESO, Pasquale, New York, NY 10011 (US); NICKELS, Michael, L., Nashville, TN 37205 (US); CROSSETTI, Henry, W., Kiawah Island, SC 29455 (US); WILCOX-ADELMAN, Sarah, Demarest, NJ 07627 (US)
(74) Representative: HGF
(86) International application number: PCT/US2020/027495
(87) International publication number: WO 2020/210509

(56) References cited:
- EP-A1- 2 392 343
- WO-A1-2019/046841
- WO-A2-02/02061
- GB-A- 2 289 841
- US-A1- 2001 006 624
- US-A1- 2002 182 267
- US-A1- 2006 193 790
- US-A1- 2014 341 819

## Description

### BACKGROUND OF THE INVENTION

Bacteria and other pathogens have been shown to be related to the development of dental disease, such as tooth decay, periodontitis, as well as oral malodor.

Periodontal disease is the result of the accumulation of bacteria-containing plaque on the teeth and the surrounding soft tissue. The progression of periodontal disease starts with a pellicle formation, which arises from salivary proteins that attach to the surface of the teeth. Once formed, oral bacteria start to attach to this pellicle, forming a biofilm. Biofilms are implicated in the etiopathogenesis of dental caries, periodontal disease and breath malodor. Continuous and regular disruption of these biofilms is imperative for the prevention and management of oral disease. Though uncalcified biofilms can be removed by routine oral hygiene aides or professional dental instruments, they have the potential to absorb minerals from the saliva becoming dental calculus making their removal difficult. Hence, these biofilms pose a great challenge to the dental clinician in the control and eradication of biofilm-associated diseases.

If the bacterial population is allowed to increase in this biofilm, plaque is formed. Plaque may be described as a specific but highly variable structural entity consisting of microorganisms and their products embedded in a highly organized intercellular matrix. The bacterial species associated with plaque number approximately 1,000, and are mainly facultative Streptococcus mutans, Fusobacterium and Actinobacteria. If left undisturbed for 24 to 48 hours, plaque will cause inflammation to the soft tissue surrounding the teeth, known as gingivitis. This is considered the first stage of periodontal disease. The bacteria associated with gingivitis is initially gram-positive (e.g., Streptoccocus, Actinomyces, Veillonella) and, as the condition worsens, gram-negative anaerobic pathogens.

Plaque will eventually become hard due to calcification from minerals found in saliva. This is known as tartar or calculus. Unlike plaque or biofilms, tartar cannot be removed by abrasion (brushing), but requires professional scaling for removal. Plaque and tartar, if undisturbed, accumulate under the gum tissue and their presence causes permanent destruction of the hard and soft tissue supporting the teeth, referred to as periodontitis. Bacteria associated with periodontitis include Porphyromonas gingivalis, Actinobacillus actinomycetemcomitans, Provetella intermedia, Bacteroides forsythus, Campylobacter rectus, Treponema and Eubacterium.

Tooth decay is also a concern, and is the result of a reaction of bacteria on sugars which product acids. These acids erode the tooth surface in the presence of fermentable carbohydrates, from sugars. Bacteria associated with this disease include Streptococcus mutans which initiates the lesion on the surface of the teeth, and Lactobacillus which is responsible for spread and penetration. Treatment options include hygiene instructions, routine professional cleaning, dietary modification, fluoride treatment and sealants. The highest incidence is in children and older adults. The challenge in children is establishing regular effective hygiene and controlling diet. In older adults, there is commonly a decrease in salivary flow, resulting in the reduced effect of the natural self-cleansing provided by saliva, and allowing for food and bacterial buildup at the gingival margins of the teeth. This reduction in saliva also results in a reduced pH. If the pH is reduced below 6, this will result in a change in the calcium and phosphate mineral balance in the oral cavity, and cause decalcification of tooth structure.

The build-up of gram-negative anaerobic bacteria on the dorsal posterior surface of the tongue and under the soft tissue supporting the teeth also presents problems in the form of oral malodor. These bacteria break down any sloughed cells that include sulfur-containing amino acids found in oral organic matter such as cellular debris, food particles and salivary proteins. This releases sulfides gases into the oral cavity (e.g., hydrogen sulfide, methyl mercaptan, thiols and disulfides), which constitute oral malodor, including halitosis. Approximately 20% of the population have persistent malodor following normal dental hygiene.

The primary causes of halitosis and morning breath are the putrefaction of gram-negative anaerobes and an abundance of sulfur containing amino acids in oral organic matter. During sleep, the lack of salivary flow, lower oxygen availability, and reduced action of the tongue and cheek, can enhance the action of these bacteria. Mouthwash users experience little lasting effect from such use, since the formulations act as temporary masking agents that briefly supplant the malodor with a more pleasant one. Alcohol containing mouthwashes, that irritate and thus enhance cellular sloughing, can actually make matters worse. Mints and chewing gum are also ineffective in the long term, as they provide only temporary concealment of the malodor. In addition, malodor can be present if tongue coating is not addressed. The coating is made up of sloughed epithelial cells, organic debris and salivary proteins. The undersurface of the tongue lacks oxygen exposure and promotes the growth of anaerobes. The dorsal posterior portion of the tongue is usually abraded by contact with the palate and anterior teeth. Individuals with a high palatal vault or limited tongue movement would be susceptible to coating formation. There are multiple factors which can contribute to formation of a coating besides anatomy, such as poor oral hygiene, failure to brush the tongue daily, or dry mouth (xerostomia). It is also known that postnasal drip and sinus infections add to the tongue coating.

The bacteria associated with malodor include Bacteroides melanogenica, Treponema denticola, Porphromonas gingivalis, Provatella intermedia, Bacteriodes forsythes, Fusobacterium, Pseudomonas, Citrobacter, Aeromonas, Salmonella and Escherichia coli.

One dilemma is that the bacteria which underlie periodontal disease are also present in the normal, native, flora. Thus, attempting to control these bacteria via the use of antibiotics would provide only a temporary detriment to the native flora.

Antimicrobial mouth rinses are currently commercially available. These mouth rinses contain several ingredients, including, for example, chlorhexidine gluconate (CHG), cetylpyridinium chloride (CPC) and essential oils (EO). CHG is active against a wide range of gram-positive and gram-negative microorganisms, and is believed to bind with salivary mucins, tooth structure, dental plaque, and oral soft tissues and is released slowly into the mouth, where it is further believed to inhibit adsorption of bacteria onto the teeth. CPC also binds to teeth and plaque, but to a lesser degree than CHG, and is generally less efficacious relative to CHG. Both CHG and EO penetrate plaque biofilm and produce changes in microbial cell surface morphology that alter coaggregation, recolonization, and thus survival. Long term use of these ingredients does not adversely affect the ecology of oral microflora, including microbial overgrowth, opportunistic infection, or development of microbial resistance. Nor is there any contribution to soft tissue lesions or mucosal aberrations and has no serious adverse effects on salivary flow, taste, tooth deposits, or dental restoration. Moreover, there is no known link between alcohol-containing mouth rinses and the risk of oral or pharyngeal cancer.

Effectiveness aside, these ingredients are also associated with side effects. For example, the use of CHG has been reported to be associated with tooth/tongue staining; mouth/throat irritation; dry mouth; unpleasant taste; decreased taste sensation; and mouth sores. CPC (included in commercially-available products such as Cepacol^{®} mouthwash, Scope^{®} mouthwash (which also includes domiphen bromide and denatured alcohol), and Crest Pro Health^{®} multi-protection mouthwash) has been associated with the staining of teeth and oral appliances; oral dryness; burning of the oral mucosa; and calculus formation, while EO has been associated to be associated with dry mouth; mask breath odor; and may make breath odor worse depending on alcohol content.

### BRIEF SUMMARY OF THE INVENTION

The invention generally relates to formulations for retarding the advance of, or treating, tooth decay, periodontitis (including its initial stage, gingivitis) and oral malodor, e.g., halitosis, and symptoms associated with dry mouth, methods for the preparation of these formulations, and related methods for using these formulations.

The present invention provides a formulation prepared via mixing a plurality of components to provide an aqueous solution or dispersion, the components comprising, consisting essentially of, or consisting of a hypobromite salt, a chlorite salt, water, and a pH adjusting agent, as well as one more optional components and/or ingredients as described herein, wherein the resulting formulation has a pH of from about 6 to about 10, and desirably from about 7 to about 10.

In related embodiments, the invention provides methods for reducing the advance of, or treating, tooth decay, periodontitis (including its initial stage, gingivitis) and oral malodor, e.g., halitosis, and symptoms associated with dry mouth, by applying the inventive formulations onto the teeth and/or surrounding soft tissue.

Other embodiments of the invention include methods for using the formulations described herein in ultrasonic cleaning, as a debridement solution, as well as for cleaning oral appliances, e.g., dentures, sleep apnea mouth devices.

### DETAILED DESCRIPTION OF THE INVENTION

The present invention, in certain embodiments, provides formulations, and related methods, suitable for application onto the oral mucosa and teeth wherein, after application of the formulations thereon, a reduction in the concentration of bacteria on teeth and/or on the surrounding soft tissue is provided. This reduction in bacteria is believed to retard the development of, or treat existing, conditions such as tooth decay, periodontitis (including gingivitis) and/or oral malodor, e.g., halitosis, and alleviate the symptoms resulting from a decrease in saliva, mucocitis and undesirable reduction in pH.

The formulations are prepared via mixing a plurality of components to provide an aqueous solution or dispersion, the components comprising, consisting essentially of, or consisting of, a hypobromite salt, a chlorite salt, water, and a pH adjusting agent, and desirably hyaluronan, as well as one more optional components and/or ingredients as described herein, wherein the resulting formulations have a pH of from about 6 to about 10, and if for application onto oral mucosa, desirably are non-acidic, having a pH of from 7 to about 10.

As certain formulations are intended for application onto the oral mucosa, each ingredient used to prepare the formulation, and the resulting formulation itself, should be non-toxic, or include ingredients at concentrations that are deemed to be non-toxic, by relevant regulatory authorities.

The formulations of the present invention were found to provide various advantages relative to existing formulations. By way of example, it was discovered that certain embodiments of the formulations are effective in reducing bacterial count upon contact with the oral mucosa (e.g., desirably possessing an oxidation reduction potential (ORP) of at least about 700 mV), while other embodiments are effective in reducing bacterial count on oral appliances (e.g., desirably possessing an ORP of at least about 650 mV) despite using relatively low concentrations of chlorite salt and hypobromite salt. Further, and desirably, certain embodiments of the formulations provide this antibacterial activity at a non-acidic pH, e.g., from 7 to about 10. Providing this degree of antibacterial activity at a non-acidic pH is highly desirable when the formulations are used to contact oral mucosa, and particularly when they are used to treat individuals suffering from periodontal disease and tooth decay as acidic liquids exacerbate this condition and associated discomfort.

It was further discovered that the formulations retain their activity (e.g., ORP) for an extended period of time, e.g., for up to about 7, 14, 30, 45, 60, 90, 120, 150 or 180 days, even in formulations that are prepared and packaged at a non-acidic pH. Desirably, and to enhance activity retention, the formulations may be packaged within a UV-resistant container, e.g., a tinted or opaque container, re-sealable if desired. In addition, it is believed that the hypobromite salt is stabilized in the formulations, even at non-acidic pHs, wherein there exists relatively minimal degradation of the hypobromite salt into a bromide and/or bromate salt after formulation preparation. This stability is desirably also retained at temperatures ranging from about 4°C to about 25°C with refrigeration assisting in enhancing stability. When packaged, it is further desirable for any headspace in the container to be filled with an inert gas (e.g., nitrogen) during packaging, and more preferably under (limited) pressure, which pressure, it is believed, will assist in retaining the chlorine dioxide in solution prior to initial use. Packaging of the formulation for single use (unit dose packaging) is also contemplated.

Each of the inventive aqueous formulations is prepared with a hypobromite salt. This salt may include one of a variety of cations, e.g., alkali and alkali earth metals salts such as lithium, sodium, potassium, calcium, magnesium and zinc, which cation is desirably a sodium and/or potassium salt, and is more desirably the sodium salt. In preparing the formulations, the hypobromite salt may be present in the aqueous dispersion or solution in weight amounts ranging from about 1, about 50, about 100, about 250, about 500, about 750, about 1,000, about 1,250, about 1,500, about 1,750 or about 2,000 ppm to about 3,000, about 5,000, about 10,000, about 15,000, about 20,000, about 25,000, about 30,000, about 40,000, about 50,000, about 60,000, about 70,000, about 80,000, about 90,000 or about 100,000 ppm, based on the total weight of the formulation. While not desiring to be bound to a particular theory, it is believed that inclusion of the hypobromite salt provides the formulations with a pathogenic effect on bacteria, viruses and fungi, e.g., via oxidation of the cell membrane therein. It is further believed that this oxidation causes instability and ultimately disintegration of that membrane. Also, because the formulations do not require uptake and metabolism, bacteria, viruses and fungi cannot develop a resistance to the formulations.

The formulations desirably are prepared using sodium hypobromite as the sole hypobromite salt, and more preferably as the sole bromine-containing compound. However, the formulations also may be prepared by optionally adding other bromine-containing compounds into the water during its preparation including, for example, hypobromous acid, bromide salts such as sodium bromide, bromites, bromates, and bromous acid, although these exemplified compounds (other than the bromide) are relatively unstable in aqueous formulations. When included in addition to the hypobromite salt, the other bromine-containing compounds are present in an amount less than the amount (by weight) of the hypobromite salt, and are desirably limited to from about 0.01, about 0.1, about 1, about 5, about 10, or about 50 ppm to no more than about 300, about 200, about 100, about 50, about 10, about 5, about 1 ppm or about 0.1 ppm, based on the weight of the formulation.

Each of the inventive aqueous formulations also is prepared with a chlorite salt. This salt may include one of a variety of cations, e.g., alkali and alkali earth metal salts, which cation is desirably a sodium salt. In preparing the formulations, the chlorite salt may be present in the formulation in weight amounts ranging from about 1, about 100, about 500, about 1,000, about 2,000, about 4,000, about 5,000, about 7,000, about 10,000, about 15,000 or about 20,000 ppm to about 30,000, about 40,000, about 50,000, about 60,000, about 70,000, about 80,000, about 90,000, about 100,000, about 120,000 or about 150,000 ppm, based on the total weight of the formulation. In the inventive formulations, and as part of the numerous reactions that occur therein, at least a portion of the chlorite salt will be subject to a reaction that provides chlorine dioxide, as well as, if sodium chlorite is used, sodium chloride. The sodium chloride is believed to catalyze the formation of a hypochlorite, which in turn enhances the production of chlorine dioxide, the latter which is desirably present in the formulation when used.

The formulations desirably are prepared using sodium chlorite as the sole chlorite, and more preferably as the sole chlorine-containing compound. However, the formulations also may be prepared by adding other optional chlorine-containing compounds into the water during its preparation including, for example, sodium hypochlorite, but desirably excludes chlorine per se and hypochlorites. The inclusion of sodium hypochlorite in particular should be limited as described herein, as it adversely affects formulation taste and odor. When included in addition to the chlorite salt, the other chlorine-containing compounds are added (when preparing the formulation) in an amount less than the amount (by weight) of the chlorite salt, and are desirably limited to from about 0.01, about 0.1, about 1, about 5, about 10, or about 50 ppm to no more than about 300, about 200, about 100, about 50, about 10, about 5, about 1 ppm, or about 0.1 ppm.

As discussed herein, the pH of the formulations should be carefully controlled. There are a variety of reasons for controlling the pH. For example, the pH of the formulations will affect the conversion of the chlorite salt, and, if included, sodium hypochlorite, to chlorine dioxide. The pH also will affect the stability of the hypobromite salt, wherein a relatively higher pH within the ranges described herein is more desirable. In this regard, the pH of the formulations ranges from about 6 to about 10, and in certain embodiments is not acidic and may range from about 7 to about 7.5, 8, 8.5, 9 or 9.5 or 10, even more desirably from about 7.5 to about 8, 8.5 or 9, preferably from about 7.5 or 8 to about 8.5, and more preferably about 8.

The pH of the formulation is provided via the inclusion of a pH adjusting agent. This agent, added to the water during preparation of the formulation, may be any suitable non-toxic ingredient, or combination of ingredients, capable of providing the desired pH in the formulations when included in a particular amount. This being said, the direct addition of acids as a means of controlling pH is not preferred, because this can adversely affect the formation of chlorine dioxide. Thus, and preferably, the pH adjusting agent is a buffer, prepared using, e.g., sodium citrate, sodium carbonate, citric acid, and will be included in an amount sufficient to provide the formulation with the desired pH. As the pH in the oral cavity will change with different stages of disease, the formulation, to be most effective, should provide antimicrobial, antiviral, and antifungal activity on the teeth and surrounding soft tissues over a range of pH values, and desirably at non-acidic pH levels.

The formulations described herein are also stable, retaining their effectiveness in reducing the quantity of bacteria, viruses and/or fungi on teeth and/or in the surrounding soft tissues for at least about 7 days, more desirably for at least about 14 days, and even more desirably for at least about 30, 45, 60, 90, 120, 150 or 180 days.

In a preferred embodiment, the formulation is an aqueous solution or dispersion comprising, consisting essentially of, or consisting of sodium hypobromite in a weight amount ranging from about 1,000, about 1,250, about 1,500, about 1,750 or about 2,000 ppm to about 3,000, about 5,000, about 10,000, about 15,000, about 20,000, about 25,000, about 30,000, about 40,000, about 50,000 or about 80,000 ppm; sodium chlorite in an amount ranging from about 5,000, about 7,000, about 10,000, about 15,000 or about 20,000 ppm to about 50,000, about 60,000, about 70,000, about 80,000, about 90,000 or about 100,000 ppm, all weight amounts being those used to prepare the formulation and are based on the total weight of the formulation; water; and a pH adjusting agent, wherein the resulting formulation has a pH of from about 5 to about 10, as well as one more optional components and/or ingredients as described herein (e.g., hyaluronan), and wherein desirably the formulation excludes various other components as described herein.

On a weight ratio basis, certain embodiments of the formulations may be prepared using chlorite to hypobromite in a ratio ranging from about 5:1 to about 1:1, and more desirably from about 4:1 to about 1.5:1.

The amount of the hypobromite and chlorite ions in the formulations after their preparation may be determined via any suitable analytical protocol, e.g., spectrophotometric, ion chromatography, HPLC, ICP and wet chemistry methods. Illustrative of one such protocol for determination of the amount of hypobromite ion via wet chemistry (and, thus, sodium hypobromite) in a solution is as follows. In a first titration (Titration I), a known volume (4 ml) of a solution prepared from sodium hypobromite (and, if desired, sodium bromate) is provided. 25 ml of deionized water (DI) is added to the 4ml solution and mixed. The resulting 29 ml of solution is titrated with 0.02M Na₂S₂O₃ (thiosulfate) until the solution turns yellow. 5 ml of a starch solution is then added, and titration is continued in the blue-colored solution until the blue-coloration disappears. In a second titration (Titration II), 4 ml of the same solution prepared from sodium hypobromite as used in Titration I is mixed with 6 ml of DI water. 1 g of ammonium sulfate is added to this mixture, which is then allowed to sit for 15 minutes. Thereafter, 3 g of KI and 10 ml of 2M H₂SO₄ are added, and the resulting mixture is allowed to sit for 10 mins. Titration is then conducted according to Titration I. The following formula allows for the calculation of the amount of hypobromite ion in the solution: Weight % hypobromite = (0.59445)(10/volume)(Titrate I volume - Titrate II volume)(Thiosulfate)(Molarity).

The formulations may desirably exclude various components, e.g., there is desirably no more than 1 ppm, more desirably no more than 0.1 ppm, even more desirably no more than 0.01 ppm, preferably no more than 0.001 ppm, and more preferably no detectable amount, based on the weight of the formulation, of these components. Illustrative of one class of components that are desirably excluded included certain components that can be oxidized, because the formulations contain relatively strong chemical oxidizers. Examples of these components include, e.g., chlorhexidine gluconate, EDTA, quaternary ammonium compounds (e.g., cetylpyridinium chloride (CPC)), CHG, certain surfactants, emollients, alcohols and essential oils. Although some quaternary ammonium compounds do possess antiseptic properties, e.g., CPC, they are not essential for the effectiveness of the formulation described herein. In this regard, a mouth rinse containing CPC (0.07%, Crest^{®} Pro-Health) as the active ingredient was found to not provide any statistically significant benefit over a mouth rinse which includes essential oils (Listerine^{®}) as the active ingredient. See Albert-Kiszely et al., "Comparison of the Effects of Cetylpyridinium Chloride with an Essential Oil Mouth Rinse on Dental Plaque and Gingivitis - a Six-month Randomized Controlled Clinical Trial," J. Clin. Periodontol. 34(8): 658-67 (Aug, 2007). Hydrogen peroxide also should be excluded because it will cause the hypobromite salt to convert to (toxic) bromine, as well as the conversion of chlorine dioxide and/or sodium chlorite to chlorine.

A further ingredient desirably included certain embodiments of the invention, and desirably in formulations that will contact the oral mucosa, is hyaluronan. Hyaluronan is hygroscopic and viscoelastic, increases the viscosity of the formulations, and is believed to provide an enhancement to the antimicrobial properties of the formulations, as well as an antiinflammatory relative to oral mucosa. The latter effect is especially desirable when the formulations are applied onto oral mucosa afflicted with periodontal disease.

Hyaluronan is polymeric, and may be used in crosslinked or non-crosslinked form. Certain hyaluronans, i.e., relatively low molecular weight hyaluronans, may elicit an inflammatory response in tissue onto which it is applied. It is therefore believed to be desirable to utilize non-crosslinked hyaluronan having a (weight average) molecular weight (MW) greater than about 500,000, desirably from about 1 million (M) to about 6M, and more desirably from about 1.5 M to about 4M. When using crosslinked hyaluronan, relatively lower molecular weight polymers may be used (e.g., less than 500,000 MW), as the crosslinking compensates for the disadvantages associated with the low molecular weight polymers.

When included, hyaluronan may be added in the formulations in an amount ranging from about 100, 200, 300, 500, 1000, 1500 or 2000 ppm to about 3000, 4000, 6000, 8000, 12000 or 16000 ppm, based on the final formulation.

Optional formulation components, one or more of which may be included in any embodiment of the invention described herein include preservatives, flavorings, sweeteners, teeth brighteners/whiteners, fluoride salts (for tooth conditioning) saliva enhancers, surfactants, cannabidiol (CBD) oil, and viscosity enhancers.

A preservative, as its name implies, is believed to assist in preserving the formulation over time by assisting in stabilizing the active ingredients, and possibly by interacting with the buffer. The preservative may be added to the formulation in any amount suitable to provide for the desired degree of preservation, and may be added in amounts ranging from about 1 ppm, about 100, about 500, about 1,000, about 2,500, about 5,000 to about 10,000, about 12,500, about 15,000, about 17,500, about 20,000, about 25,000 or about 30,000 ppm, based on the weight of the formulation. Suitable preservatives include BHT, BHA, sodium nitrate, sulfites and sodium benzoate and other food-safe preservatives, with sodium benzoate being preferred.

Flavorings (e.g., fruit or other natural flavors) and artificial sweeteners (sugars, which promote decay, should be omitted) may be added to enhance acceptance. While amounts used may vary, the amount of flavoring may range from about 0.001 wt.% to about 1 wt.%, and desirably from about 0.01 wt.% to about 0.5 wt.%, of the formulation, and the amount of artificial sweetener may range from about 0.001 wt.% to about 0.1 wt.%, and desirably from about 0.01 wt.% and about 0.5 wt.%, based on the weight of the formulation. Teeth brighteners/whiteners, e.g., silica, as well as saliva enhancers. When included, teeth brighteners may range from about 0.001 wt.% to about 1 wt.% based on the weight of the formulation, saliva enhancers may range from about 0.001 wt.% to about 1 wt.% based on the weight of the formulation.

Viscosity enhancers (in addition to, or in place of hyaluronan which provides the formulations in which it is included with a relative increase in viscosity) may be included in amounts sufficient to provide the formulations with a viscosity that may range from about 1 to about 50 centipoise (cP), desirably from about 1 to about 20 cP, and most desirably from about 1 to about 10 cP. While a relatively high viscosity formulation, e.g., about 20 to about 50 cP, may be desirable depending on the method of application to permit ease of application onto the oral surfaces, e.g., directly from a dispenser, a relatively low viscosity formulation, e.g., from about 1 to about 20 cP is desirable if the formulation is applied using an absorbent applicator, such as a cotton or synthetic swab, or used as an oral rinse, debridement solution, or appliance cleaner. The viscosity of the formulations may be determined using a Brookfield DV2T rotational viscometer at 25°C, 60 rpm, LV #3 spindle, and a 600 mL low form Griffin beaker (or equivalent container with a diameter of 8.25 cm).

Surfactants also may be desirably included in certain embodiments of the inventive formulations, and preferably in formulations useful for cleaning oral appliances. Any surfactant compatible with the formulations may be used, e.g., anionic, nonionic and cationic surfactants, with anionic surfactants being preferred, e.g., sodium lauryl sulfate. The surfactants may be included in any amount suitable to enhance the desired degree of cleansing, and may be added in amounts ranging from about 1 ppm, about 10, about 25, about 40 or about 50 to about 100, about 150, about 200, about 300, or about 500 ppm, based on the formulation.

The formulations also may optionally include glycerin. Glycerin is believed to assist in assisting solubilization and/or emulsification of any organic ingredients in the aqueous formulations, and may also provide a degree of sweetness as well as (limited) antimicrobial properties to the formulation. When included in the formulations, glycerin may be introduced in any suitable amount, ranging from about 500, about 1000, about 1200, about 2000, about 2500, about 3000, about 4000, or about 6000 to about 7000, about 10000, about 15000, about 25000, about 40000 or about 50000 ppm, based on the formulation.

The formulations may be prepared in any suitable manner. In one embodiment, the formulations may be prepared by providing: an aqueous solution of the pH adjusting agent (if a buffer, at least the acidic component); an aqueous solution of the hypobromite salt (and, if desired, and a buffer is used, a conjugate base); and an aqueous solution of the chlorite salt. The water used is preferably deionized. The aqueous solution formed by the addition of the pH adjusting agent is added to the aqueous solution formed by the addition of the chlorite salt, with the resulting aqueous solution (having a pH desirably between 5 to 7, and preferably 7 to 9) added to the solution formed by the addition of the hypobromite salt. In another embodiment, the formulation may be prepared, and packaged, in two parts and mixed prior to use to provide for activation of the formulation. In this embodiment, the aqueous solutions formed by the addition of the hypobromite and the chlorite salts are combined into one composition, with the aqueous solution formed by the addition of the pH adjusting agent (if a buffer, at least the acidic portion thereof) being added thereto prior to use.

The formulations, when applied onto at least one tooth in the oral mucosa, is effective in reducing (relative to the quantity prior to application) the quantity of plaque thereon, and also provides activity against (reduces the quantity of, after application) the primary decalcification of hydroxyapatite by the following oral bacteria: Streptococcus mutans, Fusobacterium and/or Actinobacteria.

In addition, the formulations provide a method for retarding the advancement of, or treating, periodontitis, and also reduces the quantity (relative to the quantity prior to application) of Porphyromonas gingivalis, Actinobacillus actinomycetemcomitans, Provetella intermedia, Bacteroides forsythus, Campylobacter rectus, Treponema and/or Eubacterium therein.

The formulations further provide a method for retarding the advancement of, or treating, tooth decay by reducing the quantity of Streptococcus mutans and/or Lactobacillus after application onto the affected, decayed, areas, and elevating the pH.

Moreover, malodor may also be reduced by application of the formulations into the oral cavity, this application reducing the quantity of (relative to the quantity prior to application) Bacteroides melanogenica, Treponema denticola, Porphromonas gingivalis, Provatella intermedia, Bacteriodes forsythes, Fusobacterium, Pseudomonas, Citrobacter, Aeromonas, Salmonella and/or Escherichia coli. The formulations, when introduced into the oral cavity in an effective amount (e.g., from about 1 to about 20 ml, and desirably from about 2 to about 15 ml) and retained therein for between about 1 second to about 1 minute, and more desirably from about 5 to about 30 seconds, will provide relief from malodor for at least about 4, 6, 8, 10 and preferably about 12 hours after application. It is believed that the formulations oxidize the sulfide gases in the oral cavity which reduces malodor, and also kill bacteria present in the oral cavity on contact.

In using the formulations for reducing the advancement of, or treating, plaque, periodontitis and/or tooth decay in connection with an office visit, the formulations may be applied directly into the oral cavity, onto the teeth and surrounding soft tissue (e.g., gums) and any other desired areas by any suitable means, e.g., swishing the formulation within the mouth, via spray, soft applicator, smeared (if in the form of a thickened gel, dispersion or viscous solution), so as to cover the aforementioned surfaces in a gentle manner. If deemed desirable to use the formulations on an out-patient basis, the formulation should be applied at least once per day, and may be applied twice per day, e.g., every 8-12 hours per 24-hour period. The quantity applied may vary depending on the size of the oral cavity, but generally should be sufficient to permit the formulations to completely contact all teeth and surrounding soft tissue associated with the potential development of, or existing, periodontal disease, e.g., from about 1 to about 60 ml, desirably from about 2 to about 30 ml, and more desirably from about 3 to about 20 ml, and even more desirably from about 5 to about 10 ml per treatment. For halitosis, the formulations may be applied at least, 3, 4, 5 or 6 times per day or more, depending on the severity of the condition. Because the formulations are effective on contact, the time needed for the formulations to reside on the teeth and/or surrounding soft tissue after application thereon is limited to the time required to reduce the bacterial, viral and/or fungal load to an acceptable level. Desirably, this period is from about 1 second to about two minutes, and more desirably from about 5 seconds to about 90 seconds, and more desirably from about 15 seconds to about 60 seconds, or to about 30 seconds. Repeated application (2, 3 or 4 times) within a single treatment is contemplated to maximize efficacy. Brushing with a commercial toothpaste should be delayed at least 30 minutes, and more desirably one hour, after application as the formulations continue to be effective during this post-application time period.

The formulations described herein, because of their ORP, will provide a reduction, and preferably an elimination, of bacteria within the oral cavity after application, for example, any pellicle resident on the teeth, and further delay its reformation. As a consequence, the buildup of bacteria on the tooth surface and surrounding tissue will be prevented or delayed, thereby reducing the rate of the subsequent accumulation of pathogens responsible for the conditions described herein. Generally, any additional ingredients used in the preparation of the formulations should be limited to those that do not adversely affect the oxidation potential of the formulations.

In general, the ORP of the formulations should be at least about 650 mV, and may be at least about 675, 700, 725, 750, 775, or 790 mV, but desirably should not exceed 800 mV. In one embodiment, and when the formulations are applied within the oral cavity, the ORP may range from about 700, 725 or 750 to about 800 mV, from about 725 or 750 to about 775 mV, or from about 700 or 725 to about 750 mV. In another embodiment, and when the formulations are used for cleaning oral appliances, the minimum ORP may be less than 700 mV, e.g., at least about 650 or 675 mV, as the appliance is expected to reside in the formulations for a relatively long period of time, e.g., from about 15 to about 60 mins, which permits a relatively lower ORP to be used while providing for suitable cleansing of the appliance.

The ORP of a formulation may be assessed using any suitable device, e.g., Hach Model HQ80 using a platinum probe (sensor) while the formulation is under atmospheric pressure and at room temperature, e.g., between about 20°C and about 30°C. Because the response of the sensor can degrade over time, it is desirable to test the sensor using a standard solution to verify that it is giving the correct response, such as within +/-10 mV, prior to assessing the ORP of an inventive formulation.

The formulations contemplated by the invention may vary slightly depending on their intended use. By way of illustration only, the following tables provide a description of ingredients and amounts thereof that may be used to prepare the formulations contemplated by the invention, and which may be used in the methods described herein.

Table A describes ingredients and amounts thereof that provide formulations that may be useful in the various methods contemplated by the present invention, e.g., as an oral rinse.

| Table A | Formulation 1 | | Formulation 2 | Formulation 3 | Formulation 4 |
|---|---|---|---|---|---|
| Ingredient³ | ppm | wt.% | ppm | ppm | ppm |
| DI water (1 L) | | | | | |
| Sodium chlorite | 2000 | 0.2 | 500 - 8000 | 1000 - 4000 | 1500 - 3000 |
| Sodium hypobromite | 3750 | 3.75 | 500 - 15000 | 2000 - 7500 | 3000 - 5000 |
| Glycerin | 12600 | 1.26 | 3000 - 50000 | 6000 - 25000 | 9000 - 15000 |
| Hyaluronan | 2,000 | 0.2 | 500 - 8000 | 1000 - 4000 | 1500 - 3000 |
| Sucralose | 300 | 0.03 | 50 - 1200 | 100 - 900 | 200 - 500 |
| Mint | 300 | 0.03 | 50 - 1200 | 100 - 900 | 200 - 500 |
| Sodium carbonate¹ | 1000 | 0.1 | 250 - 4000 | 500 - 2000 | 750 - 1500 |
| Citric acid¹ | 1000 | 0.1 | 250 - 4000 | 500 - 2000 | 750 - 1500 |
| Benzoic Acid² | 100 | 0.01 | 25 - 400 | 50 - 200 | 75 - 150 |
| | | | | | |
| pH | 7.5 - 8 | | 7 - 9.5 | 7 - 9 | 7 - 8.5 |

| | | | | | |
|---|---|---|---|---|---|
| ¹Amounts may vary as needed to obtain desired pH. ²Added as a preservative. ³The amount of each ingredient identified in this example (other than water, and the buffer/preservative which are already variable) may be varied ±10 wt.%. | | | | | |

Table B describes ingredients and amounts thereof that provide formulations useful in various methods contemplated by the present invention, e.g., as an enhanced oral rinse.

| Table B | Formulation 1 | | Formulation 2 | Formulation 3 | Formulation 4 |
|---|---|---|---|---|---|
| Ingredient³ | ppm | wt.% | ppm | ppm | ppm |
| DI water (1 L) | | | | | |
| Sodium chlorite | 2000 | 0.2 | 500 - 8000 | 1000 - 4000 | 1500 - 3000 |
| Sodium hypobromite | 3750 | 3.75 | 500 - 15000 | 2000 - 7500 | 3000 - 5000 |
| Glycerin | 12600 | 1.26 | 3000 - 50000 | 6000 - 25000 | 9000 - 15000 |
| Hyaluronan | 4,000 | 0.4 | 1000 - 16000 | 2000 - 12000 | 3000 - 6000 |
| Sucralose | 300 | 0.03 | 50 - 1200 | 100 - 900 | 200 - 500 |
| Mint | 300 | 0.03 | 50 - 1200 | 100 - 900 | 200 - 500 |
| Sodium carbonate¹ | 1000 | 0.1 | 250 - 4000 | 500 - 2000 | 750 - 1500 |
| Citric acid¹ | 1000 | 0.1 | 250 - 4000 | 500 - 2000 | 750 - 1500 |
| Benzoic Acid² | 100 | 0.01 | 25 - 400 | 50 - 200 | 75 - 150 |
| | | | | | |
| pH | | 7.5 - 8 | 7 - 9.5 | 7 - 9 | 7 - 8.5 |

| | | | | | |
|---|---|---|---|---|---|
| ¹Amounts may vary as needed to obtain desired pH. ²Added as a preservative. ³The amount of each ingredient identified in this example (other than water, and the buffer/preservative which are already variable) may be varied ±10 wt.%. | | | | | |

Table C describes ingredients and amounts thereof that provide formulations useful in various methods contemplated by the present invention, e.g., as a debridement solution for use in ultrasonic cleaning.

| Table C | Formulation 1 | | Formulation 2 | Formulation 3 | Formulation 4 |
|---|---|---|---|---|---|
| Ingredient³ | ppm | wt.% | ppm | ppm | ppm |
| DI water (1 L) | | | | | |
| Sodium chlorite | 2000 | 0.2 | 500 - 8000 | 1000 - 4000 | 1500 - 3000 |
| Sodium hypobromite | 3750 | 3.75 | 750 - 15000 | 2000 - 7500 | 3000 - 5000 |
| Glycerin | 12600 | 1.26 | 3000 - 50000 | 6000 - 25000 | 9000 - 15000 |
| Sucralose | 300 | 0.03 | 50 - 1200 | 100 - 900 | 200 - 500 |
| Mint | 300 | 0.03 | 50 - 1200 | 100 - 900 | 200 - 500 |
| Sodium carbonate¹ | 1000 | 0.1 | 250 - 4000 | 500 - 2000 | 750 - 1500 |
| Citric acid¹ | 1000 | 0.1 | 250 - 4000 | 500 - 2000 | 750 - 1500 |
| Benzoic Acid² | 100 | 0.01 | 25 - 400 | 50 - 200 | 75 - 150 |
| | | | | | |
| pH | 7.5 - 8.5 | | 7 - 10 | 7 - 9.5 | 7 - 9 |

| | | | | | |
|---|---|---|---|---|---|
| ¹Amounts may vary as needed to obtain desired pH. ²Added as a preservative. ³The amount of each ingredient identified in this example (other than water, and the buffer/preservative which are already variable) may be varied ±10 wt.%. | | | | | |

Table D describes ingredients and amounts thereof that provide formulations useful in various methods contemplated by the present invention, e.g., as an oral appliance cleaner.

| Table D | Formulation 1 | | Formulation 2 | Formulation 3 | Formulation 4 |
|---|---|---|---|---|---|
| Ingredient³ | ppm | wt.% | ppm | ppm | ppm |
| DI water (1 L) | | | | | |
| Sodium chlorite | 1000 | 0.1 | 250 - 4000 | 500 - 2000 | 750 - 1500 |
| Sodium hypobromite | 3750 | 3.75 | 750 - 15000 | 2000 - 7500 | 3000 - 5000 |
| Glycerin | 5000 | 0.5 | 1200 - 20000 | 2500 - 10000 | 4000 - 7000 |
| Sodium lauryl sulfate | 50 | 0.005 | 10 - 300 | 25 - 200 | 40 -100 |
| Mint | 150 | 0.015 | 50 - 700 | 75 - 500 | 100 - 300 |
| Sodium carbonate¹ | 1000 | 0.1 | 250 - 4000 | 500 - 2000 | 750 - 1500 |
| Citric acid¹ | 1000 | 0.1 | 250 - 4000 | 500 - 2000 | 750 - 1500 |
| Benzoic Acid² | 100 | 0.01 | 25 - 400 | 50 - 200 | 75 - 150 |
| | | | | | |
| pH | 7.5 - 8.5 | | 7 - 10 | 7 - 9.5 | 7 - 9 |

| | | | | | |
|---|---|---|---|---|---|
| ¹Amounts may vary as needed to obtain desired pH. ²Added as a preservative. ³The amount of each ingredient identified in this example (other than water, and the buffer/preservative which are already variable) may be varied ±10 wt.%. | | | | | |

The methods of the present invention include a method for reducing plaque which comprises applying the inventive formulation onto at least one tooth in an amount and for a time sufficient to reduce the amount of plaque thereon. An assessment of the effectiveness of the formulation in reducing plaque may be conducted via any known means, including qualitatively via the use of an oral disclosing solution. These disclosing solutions, which include a dye that adheres to plaque, may be applied before and after application of the formulation onto the tooth to be treated, with the relative intensity of the dye on the tooth being compared before and after use. A relatively lower dye intensity after application of the formulation indicates a reduction in plaque. Effectiveness also may be determined via a comparison of pre- and post-treatment saliva samples via a DNA assay for bacteria.

The invention also contemplates a method for reducing the quantity of bacteria on at least one tooth, or in areas within the oral cavity, which comprises applying the inventive formulation onto the tooth or area of the oral cavity in an amount and for a time sufficient to reduce the quantity of bacteria thereon. Regarding the effectiveness of the formulation in reducing bacteria on the teeth, and in the oral cavity, any known method may be used, e.g., an oral disclosing dye because a reduction in plaque is also indicative of a reduction in bacteria.

The invention further comprises a method for decreasing inflammation of oral soft tissues resulting from insufficient saliva. The method comprises applying the inventive formulations onto the oral soft tissues in a mouth afflicted with insufficient saliva, desirably at regular intervals, e g. once or twice daily, for at least 1, 2, 3, 7, 10, 14 or 21 days, in an amount and for a time sufficient to reduce the inflammation.

The invention also provides a method for minimizing decalcification of a tooth resulting from insufficient saliva production. The method comprises applying the inventive formulations onto a tooth suffering from decalcification resulting from insufficient saliva, desirably at regular intervals, e g. once or twice daily, in an amount and for a time sufficient to minimize tooth decalcification.

The invention further contemplates methods for oral care which include using the inventive formulations in ultrasonic cleaning, as a debridement solution. The invention also contemplates use of the formulations for cleaning oral appliances, e.g., dentures, sleep apnea mouth devices, via immersion of these devices into the formulations for up to 10, 15, 30, 60 or 120 minutes, or up to 24 hours.

The following examples are provided to illustrate the present invention, and should be understood to not limit the scope of the invention.

### EXAMPLE 1

An illustrative formulation suitable for use in ultrasonic cleaning (as a debridement solution) is provided by combining the ingredients as set forth in the following table.

| Ingredient | ppm | wt.% |
|---|---|---|
| DI water (1 L) | | |
| Sodium chlorite | 2000 | 0.20 |
| Sodium hypobromite | 3750 | 0.375 |
| Glycerin | 12600 | 1.26 |
| Sucralose | 300 | 0.03 |
| Mint | 300 | 0.030 |
| Sodium carbonate¹ | 1000 | 0.1 |
| Citric acid¹ | 1000 | 0.1 |
| Benzoic acid² | 100 | 0.01 |

| | | |
|---|---|---|
| ¹Amounts may vary as needed to obtain desired pH. ²Added as a preservative. | | |

An exemplary process for preparing 1 L of this formulation is as follows. 100 ml DI water is added to a suitable (non-reactive) container. 2 g sodium chlorite (anhydrous, ca. 98% purity) is added to the DI water, and gently mixed until dissolved. Flavoring (e.g., mint) and sweetener (e.g., sucralose, glycerin) are then added with gentle mixing until dissolved (approximately 10 minutes). If desired, the oxidation reduction potential (ORP) of this mixture may be assessed, and if so, should be no less than 700 mV. 25 ml of NaBrO (0.1N) is added to the mixture with gentle stirring. The pH then may be assessed, and should range from 7.5 to 8. A final ORP assessment should be undertaken to ensure the ORP of the mixture is at least 700 mV. The pH also may be adjusted as needed to reach an optimal value of 8, preferably via the use of a buffer solution (e.g., sodium carbonate and citric acid and/or benzoic acid). Additional DI water is then added to bring the mixture to 1 L total volume, with the product then being protected from light (e.g., amber or opaque container) until used. The amount of each ingredient identified in this example may be varied ±10 wt.%.

### EXAMPLE 2

An illustrative formulation suitable for use as an oral rinse is provided by combining the ingredients as set forth in the following table.

| Ingredient | ppm | wt.% |
|---|---|---|
| DI water (1 L) | | |
| Sodium chlorite | 2000 | 0.2 |
| Sodium hypobromite | 3750 | 3.75 |
| Glycerin | 12600 | 1.26 |
| Hyaluronan | 2,000 | 0.2 |
| Sucralose | 300 | 0.03 |
| Mint | 300 | 0.03 |
| Sodium carbonate¹ | 1000 | 0.1 |
| Citric acid¹ | 1000 | 0.1 |
| Benzoic Acid² | 100 | 0.01 |

| | | |
|---|---|---|
| ¹Amounts may vary as needed to obtain desired pH. ²Added as a preservative. | | |

An exemplary process for preparing 1L of this formulation is as follows. 500 ml DI water is added to a suitable (non-reactive) container. The hyaluronan is then added, and allowed up to 24 hours to dissolve. The use of heat, and uncoated metal stirring devices, should be avoided. Thereafter, 2 g sodium chlorite (anhydrous, ca. 98% purity) is added to the DI water, and gently mixed until dissolved. Flavoring (e.g., mint) and sweetener (e.g., sucralose, glycerin) are then added with gentle mixing until dissolved (approximately 10 minutes). If desired, the oxidation reduction potential (ORP) of this mixture may be assessed, and if so, should be no less than 700 mV. 50 ml of NaBrO (0.1N) is added to the mixture with gentle stirring. The pH then may be assessed, and should range from 7.5 to 8.5. A final ORP assessment should be undertaken to ensure the ORP is at least 700 mV. The pH may be adjusted as needed to reach an optimal value of 8 via the use of a buffer (e.g., sodium carbonate and citric acid and/or benzoic acid). Additional DI water is then added to bring the mixture to 1 L total volume, with the product then being protected from light (e.g., amber or opaque container) until used. The amount of each ingredient identified in this example may be varied ±10 wt.%.

A human subject may introduce about 10 to about 50 ml of this oral rise into his or her mouth, allow the rinse to reside in the mouth for about 30 to about 60 seconds (preferably, while swishing the rinse in the mouth), and thereafter removing the rinse. This process may be performed at least once daily, and more desirably twice daily.

### EXAMPLE 3

An illustrative enhanced formulation suitable for use as an oral rinse is provided by combining the ingredients as set forth in the following table.

| Ingredient | ppm | wt.% |
|---|---|---|
| DI water (1 L) | | |
| Sodium chlorite | 2000 | 0.2 |
| Sodium hypobromite | 3750 | 3.75 |
| Glycerin | 12600 | 1.26 |
| Hyaluronan | 4000 | 0.4 |
| Sucralose | 300 | 0.03 |
| Mint | 300 | 0.03 |
| Sodium carbonate¹ | 1000 | 0.1 |
| Citric acid¹ | 1000 | 0.1 |
| Benzoic Acid² | 100 | 0.01 |

| | | |
|---|---|---|
| ¹Amounts may vary as needed to obtain desired pH. ²Added as a preservative. | | |

An exemplary process for preparing 1L of this formulation is as follows. 500 ml DI water is added to a suitable (non-reactive) container. The hyaluronan is then added, and allowed up to 24 hours to dissolve. The use of heat, and uncoated metal stirring devices, should be avoided. Thereafter, 2 g sodium chlorite (anhydrous, ca. 98% purity) is added to the DI water, and gently mixed until dissolved. Flavoring (e.g., mint) and sweetener (e.g., sucralose, glycerin) are then added with gentle mixing until dissolved (approximately 10 minutes). If desired, the oxidation reduction potential (ORP) of this mixture may be assessed, and if so, should be no less than 700 mV. 25 to 50 ml of NaBrO (0.1N) is added to the mixture with gentle stirring. The pH then may be assessed, and should range from 7.5 to 8.5. A final ORP assessment should be undertaken to ensure the ORP is at least 700 mV. The pH also may be adjusted as needed to reach an optimal value of 8 via the use of a buffer (e.g., sodium carbonate and citric acid and/or benzoic acid). Additional DI water is then added to bring the mixture to 1 L total volume, with the product then being protected from light (e.g., amber or opaque container) until used. The amount of each ingredient identified in this example may be varied ±10 wt.%.

A human subject may introduce about 10 to about 50 ml of this oral rise into his or her mouth, allow the rinse to reside in the mouth for about 30 to about 60 seconds (preferably, while swishing the rinse in the mouth), and thereafter removing the rinse. This process may be performed at least once daily, and more desirably twice daily.

### EXAMPLE 4

An illustrative formulation suitable for use as an oral appliance cleaner is provided by combining the ingredients as set forth in the following table.

| Ingredient | ppm | wt.% |
|---|---|---|
| DI Water (1 L) | | |
| Sodium chlorite | 1000 | 0.1 |
| Sodium hypobromite | 3750 | 3.75 |
| Glycerin | 5,000 | 0.5 |
| Sodium lauryl sulfate | 50 | 0.005 |
| Mint | 150 | 0.015 |
| Sodium carbonate¹ | 1000 | 0.1 |
| Citric acid¹ | 1000 | 0.1 |
| Benzoic Acid² | 100 | 0.01 |

| | | |
|---|---|---|
| ¹Amounts may vary as needed to obtain desired pH. ²Added as a preservative. | | |

An exemplary process for preparing 1L of this formulation is as follows. 100 ml DI water is added to a suitable (non-reactive) container. 1 g sodium chlorite (anhydrous, ca. 98% purity) is added to the DI water, and gently mixed until dissolved. Glycerin and a flavoring agent (e.g., mint) is then added with gentle mixing until dissolved (approximately 10 minutes). If desired, the oxidation reduction potential (ORP) of this mixture may be assessed, and if so, should be no less than 650 mV. 25 ml of NaBrO (0.1N) is added to the mixture with gentle stirring. The pH then may be assessed, and should range from 7.5 and 8. Sodium lauryl sulfate is then added to this mixture with stirring. A final ORP assessment should be undertaken to ensure the ORP of the mixture is at least 650 mV. The pH also may be adjusted as needed to reach an optimal value of 8, preferably via the use of a buffer solution (e.g., sodium carbonate and citric acid and/or benzoic acid). Additional DI water is then added to bring the mixture to 1 L total volume, with the product then being protected from light (e.g., amber or opaque container) until used. The amount of each ingredient identified in this example may be varied ±10 wt.%.

An assessment of the effectiveness of the formulation and related methods relating to periodontitis (including gingivitis) or tooth decay may be assessed quantitatively by assessing and comparing the quantity of bacteria present on the afflicted tooth before and after application of the formulation.

Unless otherwise specified, all quantities described herein are by weight, and are based on the total weight of the formulation.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example; "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein.

## Claims

1. A formulation prepared via mixing a plurality of components to provide an aqueous solution or dispersion, the components comprising a hypobromite salt, a chlorite salt, water, and a pH adjusting agent, wherein the resulting formulation has a pH of from about 6 to about 10.

2. The formulation of claim 1, wherein the hypobromite salt and the chlorite salt are sodium salts.

3. The formulation of claim 1 or claim 2, wherein the pH adjusting agent is a buffer.

4. The formulation of any preceding claim, further comprising one or more of a preservative, a flavoring, a sweetener, a teeth brightener, a teeth whitener, a fluoride salt, a saliva enhancer, a surfactant, a cannabidiol (CBD) oil, and a viscosity enhancer.

5. The formulation of any preceding claim, further comprising a preservative selected from the group consisting of BHT, BHA, sodium nitrate, a sulfite, and sodium benzoate.

6. The formulation of any preceding claim packaged within an opaque container.

7. The formulation of any preceding claim, wherein the formulation excludes one or more of chlorhexidine gluconate, EDTA, quaternary ammonium compounds (e.g., cetylpyridinium chloride (CPC)), CHG, certain surfactants, emollients, alcohols and essential oils.

8. The formulation of any preceding claim, wherein the amount of hypobromite salt used in the preparation of the formulation is about 1 to about 10,000 ppm, and wherein the amount of chlorite salt used in the preparation of the formulation is about 1 to about 50,000 ppm based on the total weight of the formulation.

9. The formulation of claim 8, wherein the amount of hypobromite salt used in the preparation of the formulation is about 1 to about 3,000 ppm or the amount of chlorite salt used in the preparation of the formulation is about 1 to about 30,000 ppm based on the total weight of the formulation.

10. The formulation of claims 1 to 9 for use in reducing the quantity of bacteria, viruses and/or fungi on teeth and/or in the surrounding soft tissues for at least about 7 days.

11. The formulation of claims 1 to 9 for use in retarding the advance of, or treating, tooth decay, periodontitis, gingivitis, oral malodor, halitosis and symptoms associated with dry mouth.

12. The formulation for use of claim 11, wherein:
the bacteria associated with gingivitis are selected from the group consisting of: Streptoccocus, Actinomyces and Veillonella;
the bacteria associated with periodontitis are selected from the group consisting of: Porphyromonas gingivalis, Actinobacillus actinomycetemcomitans, Provetella intermedia, Bacteroides forsythus, Campylobacter rectus, Treponema and Eubacterium
the bacteria associated with oral malodor are selected from the group consisting of: Bacteroides melanogenica, Treponema denticola, Porphromonas gingivalis, Provatella intermedia, Bacteriodes forsythes, Fusobacterium, Pseudomonas, Citrobacter, Aeromonas, Salmonella and Escherichia coli;
the bacteria associated with tooth decay are selected from the group consisting of: Streptococcus mutans and Lactobacillus.

13. The formulation of claims 1 to 9 for use in reducing tooth plaque and reduces decalcification of a tooth resulting from insufficient saliva production.

14. The formulation for use of claim 13, wherein the bacteria associated tooth plaque and decalcification are selected from the group consisting of: Streptococcus mutans, Fusobacterium and Actinobacteria.

15. The formulation of claims 1 to 9 for use in decreasing inflammation of oral soft tissues resulting from insufficient saliva.

16. The formulation of claims 1 to 9 for use in medicine.

## Patentansprüche

1. Formulierung, hergestellt durch Mischen einer Vielzahl von Komponenten, um eine wässrige Lösung oder Dispersion bereitzustellen, wobei die Komponenten ein Hypobromitsalz, ein Chloritsalz, Wasser und ein pH-Einstellmittel umfassen, wobei die resultierende Formulierung einen pH-Wert von etwa 6 bis etwa 10 aufweist.

2. Formulierung nach Anspruch 1, wobei das Hypobromitsalz und das Chloritsalz Natriumsalze sind.

3. Formulierung nach Anspruch 1 oder Anspruch 2, wobei das pH-Einstellmittel ein Puffer ist.

4. Formulierung nach einem vorhergehenden Anspruch, ferner umfassend eines oder mehrere von einem Konservierungsmittel, einem Aroma, einem Süßungsmittel, einem Zahnaufheller, einem Zahnbleicher, einem Fluoridsalz, einem Speichelverstärker, einem Tensid, einem Cannabidiol(CBD)-Öl und einem Viskositätsverstärker.

5. Formulierung nach einem vorhergehenden Anspruch, ferner umfassend ein Konservierungsmittel, ausgewählt aus der Gruppe bestehend aus BHT, BHA, Natriumnitrat, einem Sulfit und Natriumbenzoat.

6. Formulierung nach einem vorhergehenden Anspruch, verpackt in einem opaken Behälter.

7. Formulierung nach einem vorhergehenden Anspruch, wobei die Formulierung eines oder mehrere von Chlorhexidingluconat, EDTA, quaternären Ammoniumverbindungen (z. B. Cetylpyridiniumchlorid (CPC)), CHG, bestimmten Tensiden, Weichmachern, Alkoholen und ätherischen Ölen nicht enthält.

8. Formulierung nach einem vorhergehenden Anspruch, wobei die Menge an Hypobromitsalz, die bei der Herstellung der Formulierung verwendet wird, etwa 1 bis etwa 10.000 ppm beträgt, und wobei die Menge an Chloritsalz, die bei der Herstellung der Formulierung verwendet wird, etwa 1 bis etwa 50.000 ppm beträgt, bezogen auf das Gesamtgewicht der Formulierung.

9. Formulierung nach Anspruch 8, wobei die Menge an Hypobromitsalz, die bei der Herstellung der Formulierung verwendet wird, etwa 1 bis etwa 3.000 ppm beträgt oder die Menge an Chloritsalz, die bei der Herstellung der Formulierung verwendet wird, etwa 1 bis etwa 30.000 ppm beträgt, bezogen auf das Gesamtgewicht der Formulierung.

10. Formulierung nach den Ansprüchen 1 bis 9 zur Verwendung beim Reduzieren der Menge an Bakterien, Viren und/oder Pilzen auf Zähnen und/oder in den umgebenden Weichgeweben für mindestens etwa 7 Tage.

11. Formulierung nach den Ansprüchen 1 bis 9 zur Verwendung bei der Verzögerung des Fortschreitens oder der Behandlung von Karies, Parodontitis, Gingivitis, Mundgeruch, Halitosis und Symptomen im Zusammenhang mit trockenem Mund.

12. Formulierung zur Verwendung nach Anspruch 11, wobei:
die mit Gingivitis im Zusammenhang stehenden Bakterien aus der Gruppe ausgewählt sind, die aus Folgendem besteht: Streptococcus, Actinomyces und Veillonella;
die mit Parodontitis im Zusammenhang stehenden Bakterien aus der Gruppe ausgewählt sind, die aus Folgendem besteht: Porphyromonas gingivalis, Actinobacillus actinomycetemcomitans, Provetella intermedia, Bacteroides forsythus, Campylobacter rectus, Treponema und Eubacterium,
die mit Mundgeruch im Zusammenhang stehenden Bakterien aus der Gruppe ausgewählt sind, die aus Folgendem besteht: Bacteroides melanogenica, Treponema denticola, Porphromonas gingivalis, Provatella intermedia, Bacteriodes forsythes, Fusobacterium, Pseudomonas, Citrobacter, Aeromonas, Salmonella und Escherichia coli;
die mit Karies im Zusammenhang stehenden Bakterien aus der Gruppe ausgewählt sind, die aus Folgendem besteht: Streptococcus mutans und Lactobacillus.

13. Formulierung nach den Ansprüchen 1 bis 9 zur Verwendung bei der Reduzierung von Zahnbelag, und die eine Entkalkung eines Zahns aufgrund unzureichender Speichelproduktion reduziert.

14. Formulierung zur Verwendung nach Anspruch 13, wobei die mit Zahnbelag und Entkalkung im Zusammenhang stehenden Bakterien aus der Gruppe ausgewählt sind, die aus Folgendem besteht: Streptococcus mutans, Fusobacterium und Actinobacteria.

15. Formulierung nach den Ansprüchen 1 bis 9 zur Verwendung bei der Verringerung einer Entzündung von oralen Weichgeweben aufgrund von unzureichendem Speichel.

16. Formulierung nach den Ansprüchen 1 bis 9 zur Verwendung in der Medizin.

## Revendications

1. Formulation préparée en mélangeant une pluralité de composants pour obtenir une solution ou une dispersion aqueuse, les composants comprenant un sel d'hypobromite, un sel de chlorite, de l'eau et un agent d'ajustement du pH, la formulation résultante ayant un pH d'environ 6 à environ 10.

2. Formulation selon la revendication 1, dans laquelle le sel d'hypobromite et le sel de chlorite sont des sels de sodium.

3. Formulation selon la revendication 1 ou la revendication 2, dans laquelle l'agent d'ajustement du pH est un tampon.

4. Formulation selon l'une quelconque des revendications précédentes, comprenant en outre un ou plusieurs parmi un conservateur, un arôme, un édulcorant, un azurant dentaire, un blanchisseur dentaire, un sel de fluorure, un activateur de salive, un tensioactif, une huile de cannabidiol (CBD) et un activateur de viscosité.

5. Formulation selon l'une quelconque des revendications précédentes, comprenant en outre un conservateur choisi dans le groupe constitué de BHT, BHA, nitrate de sodium, sulfite et benzoate de sodium.

6. Formulation selon l'une quelconque des revendications précédentes, conditionnée dans un récipient opaque.

7. Formulation selon l'une quelconque des revendications précédentes, ladite formulation excluant un ou plusieurs parmi gluconate de chlorhexidine, EDTA, composés d'ammonium quaternaire (par exemple, chlorure de cétylpyridinium (CPC)), CHG, certains tensioactifs, émollients, alcools et huiles essentielles.

8. Formulation selon l'une quelconque des revendications précédentes, dans laquelle la quantité de sel d'hypobromite utilisée dans la préparation de la formulation est d'environ 1 à environ 10 000 ppm, et dans laquelle la quantité de sel de chlorite utilisée dans la préparation de la formulation est d'environ 1 à environ 50 000 ppm sur la base du poids total de la formulation.

9. Formulation selon la revendication 8, dans laquelle la quantité de sel d'hypobromite utilisée dans la préparation de la formulation est d'environ 1 à environ 3 000 ppm ou la quantité de sel de chlorite utilisée dans la préparation de la formulation est d'environ 1 à environ 30 000 ppm sur la base du poids total de la formulation.

10. Formulation selon les revendications 1 à 9 destinée à être utilisée pour réduire la quantité de bactéries, de virus et/ou de champignons sur les dents et/ou dans les tissus mous environnants pendant au moins environ 7 jours.

11. Formulation selon les revendications 1 à 9 destinée à être utilisée pour retarder l'avancement ou le traitement des caries dentaires, de la parodontite, de la gingivite, de la mauvaise haleine, de l'halitose et des symptômes associés à la sécheresse buccale.

12. Formulation destinée à être utilisée selon la revendication 11, dans laquelle :
les bactéries associées à la gingivite sont choisies dans le groupe constitué de : Streptocoque, Actinomyces et Veillonella ;
les bactéries associées à la parodontite sont choisies dans le groupe constitué de : Porphyromonas gingivalis, Actinobacillus actinomycetemcomitans, Provetella intermedia, Bacteroides forsythus, Campylobacter rectus, Treponema et Eubacterium
les bactéries associées à la mauvaise haleine sont choisies dans le groupe constitué de : Bacteroides melanogenica, Treponema denticola, Porphromonas gingivalis, Provatella intermedia, Bacteriodes forsythes, Fusobacterium, Pseudomonas, Citrobacter, Aeromonas, Salmonella et Escherichia coli ;
les bactéries associées aux caries dentaires sont choisies dans le groupe constitué de : Streptococcus mutans et Lactobacillus.

13. Formulation selon les revendications 1 à 9 destinée à être utilisée pour réduire la plaque dentaire et qui réduit la décalcification d'une dent résultant d'une production insuffisante de salive.

14. Formulation destinée à être utilisée selon la revendication 13, dans laquelle la plaque dentaire et la décalcification associées aux bactéries sont choisies dans le groupe constitué de : Streptococcus mutans, Fusobacterium et Actinobacteria.

15. Formulation selon les revendications 1 à 9 destinée à être utilisée pour réduire l'inflammation des tissus mous buccaux résultant d'une insuffisance de salive.

16. Formulation selon les revendications 1 à 9 destinée à être utilisée en médecine.
